# EUROPEAN PATENT APPLICATION

(11) **EP 3 639 816 A1**
(43) Date of publication of application: **22.04.2020**
(21) Application number: 18200743.5
(22) Date of filing: 16.10.2018
(51) Int. Cl.: A61K 31/12, A61K 31/136, A61K 31/357, A61K 31/404, A61K 45/06, A61K 9/00, A61P 11/00, A61P 31/00, A61P 31/02, A61P 31/04, A61P 43/00

(54) **COMPOUNDS FOR USE IN THE TREATMENT OF BACTERIAL INFECTIONS**

(71) Applicant: Universität Konstanz, 78464 Konstanz (DE)
(72) Inventor: BÖTTCHER, Thomas, 78467 Konstanz (DE); PROTHIWA, Michaela, 78462 Konstanz (DE); ENGLMAIER, Felix, 30177 Hannover (DE)
(74) Representative: Müller-Boré & Partner Patentanwälte PartG mbB

(57) **Abstract**

The present invention relates to a compound which can be used in the treatment of infections with pathogenic bacteria, in particular pathogens mediating pathogenicity by quinolone dependent production of virulence factors, such as e.g. *Pseudomonas aeruginosa* and species of the genus *Burkholderia*, and which can be used for treating bacterial or chronic infections or can be used in combination with other anti-bacterial agents, such as antibiotics, to increase sensitivity of the bacteria for treatment of e.g. multiresistant strains in e.g. mammals.

## Description

The present invention relates to a compound which can be used in the treatment of infections with pathogenic bacteria, in particular pathogens mediating pathogenicity by quinolone dependent production of virulence factors, such as e.g. *Pseudomonas aeruginosa* and species of the genus *Burkholderia,* and which can be used for treating bacterial or chronic infections or can be used in combination with other anti-bacterial agents, such as antibiotics, to increase sensitivity of the bacteria for treatment of e.g. multiresistant strains in e.g. mammals.

Due to the rising threat of multidrug resistant bacteria, alternative strategies to treat bacterial infections are desirable and much needed. Possible alternatives include anti-virulence drugs that do not kill bacteria but disarm them and reduce the production of toxins and factors which impair the immune response or promote infection and colonization of the host organism.

Examples of these bacteria with multidrug resistance are represented by *Pseudomonas aeruginosa* and species of the genus *Burkholderia* which are important Gram-negative pathogens causing many severe and life-threatening infections including but not limited to sepsis, wound infections, endocarditis, meningitis, and chronic respiratory infections in cystic fibrosis patients. The virulence of *P*. *aeruginosa* and species of the genus *Burkholderia* are mediated by quinolone signal dependent production of virulence factors as well as quinolones as toxicants and agents that lead to impairment of the immune response or allow competition with commensal bacteria and are thus important for the colonization of the host.

Thus, the technical problem underlying the present invention is to provide a compound, which can be used in the treatment of infections with pathogenic bacteria, in particular pathogens mediating pathogenicity by quinolone dependent production of virulence factors, such as e.g. *Pseudomonas aeruginosa* and species of the genus *Burkholderia,* preferably in the treatment of infections with pathogenic multi-resistant species, as well as a corresponding pharmaceutical composition.

The solution to the above technical problem is achieved by the embodiments characterized in the claims.

In particular, the present invention relates to a compound for use in the treatment of infections with pathogenic bacteria, in particular pathogens mediating pathogenicity by quinolone dependent production of virulence factors, such as e.g. *Pseudomonas aeruginosa* and species of the genus *Burkholderia*, in vertebrates such as mammals, particularly humans, wherein the compound is represented by the general Formula (1) or a pharmaceutically acceptable salt thereof 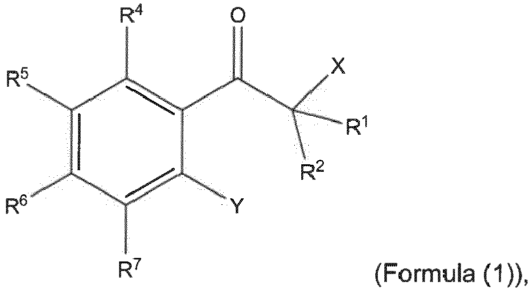 wherein
X is a halogen atom;
Y is NHR³, a hydrogen atom, or a halogen atom;
R¹ and R² are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group;
R³, when present, is selected from the group consisting of a hydrogen atom, a substituted or unsubstituted alkyl group having from 1 to 11 carbon atoms, a substituted or unsubstituted cycloalkyl group having from 4 to 12 carbon atoms, a substituted or unsubstituted alkenyl group having from 2 to 11 carbon atoms, a substituted or unsubstituted cycloalkenyl group having from 4 to 12 carbon atoms, a substituted or unsubstituted alkynyl group having from 2 to 11 carbon atoms, a substituted or unsubstituted aryl group, and a substituted or unsubstituted heteroaryl group, wherein R³ may bind to R⁷ to form a ring; and
R⁴ to R⁷ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, a halogen atom, -NE¹E², -NO₂, -CN, -OE³, -SE⁴, -C(O)E⁵, -C(O)NE⁶E⁷, -COOE⁸, and -SO₃E⁹, wherein E¹ to E⁹ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, and wherein two or more of R⁴ to R⁷ may bind to each other to form one or more rings.

The compound for use according to the present invention is preferably able to reduce the pathogenicity of pathogenic bacteria mediating pathogenicity by quinolone dependent production of virulence factors, such as e.g. *Pseudomonas aeruginosa* and species of the genus *Burkholderia.*

The pathogenicity of *P. aeruginosa* is mediated by its enormous arsenal of virulence factors which is orchestrated by the population density-dependent *pqs* quorum sensing system. The signalling molecules hereby are the Pseudomonas quinolone signal (PQS) as well as the biosynthetic precursor 2-heptyl-4-quinolone (HHQ) and congeners with different alkyl chain length and degree of saturation. These signals that are important for the full virulence of *P*. *aeruginosa* are produced via the *pqsABCDE* gene cluster and *pqsH.* PQS and HHQ are not only important for coordinating virulence factor production of *P. aeruginosa* but these quinolone have also a role in modulating and suppressing the human immune response. The same gene cluster is also responsible for the biosynthesis of 2-alkyl-4-quinolone *N*-oxides (AQNOs) which are important antibacterial compounds that help *P*. *aeruginosa* to defend and occupy its niches in the human body.

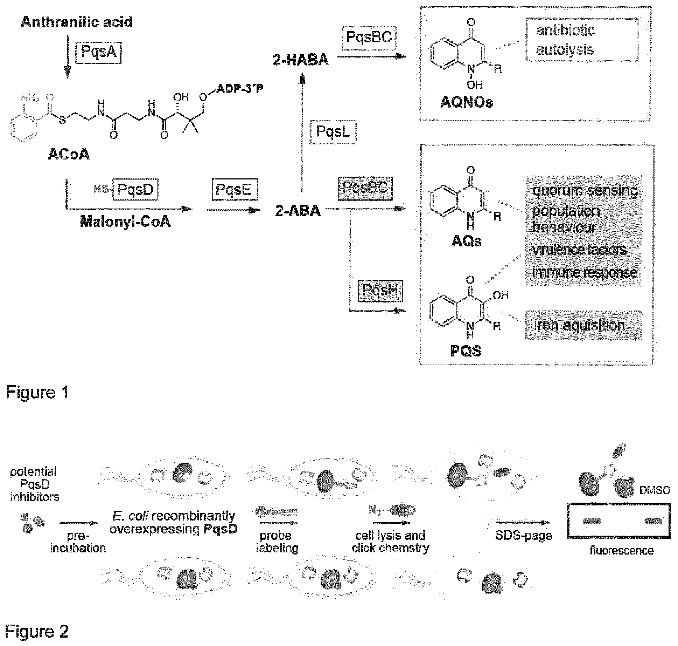

A common step in the biosynthesis of all quinolones is the decarboxylative coupling reaction of coenzyme A-activated anthranilic acid (ACoA) with malonyl-CoA, which is catalysed by the enzyme PqsD. The active site of PqsD comprises a nucleophilic cysteine residue, which is involved in the transfer of the anthraniloyl moiety. Hydrolysis of the resulting thioester by enzyme PqsE leads to 2-aminobenzoylacetate (2-ABA), which is the precursor for the subsequent reactions to 2-alkyl-4-quinolones (AQs), 3-hydroxy-AQs, and AQNOs (Figure 1). The homolog to PqsD in species of the genus *Burkholderia* is the enzyme HmqD.

Quinolone biosynthesis is thus highly important for the pathogenicity of *P. aeruginosa* and of species of the genus *Burkholderia.* Although many inhibitors of the quinolone biosynthesis showed great potency *in vitro* in enzyme-based assays, they were only poorly active in live cells of *P*. *aeruginosa* and only incompletely inhibited quinolone biosynthesis (20-60%) at very high concentrations (>200 µM), which are not to be considered relevant for further medicinal development.

However, the compound for use according to the present invention is advantageously able to not only suppress quinolone biosynthesis *in vitro,* but preferably also to inhibit quinolone biosynthesis, e.g. via PqsD in PqsD expressing live-cells, such as modified *E. coli* and *P*. *aeruginosa,* and via HmqD in species of the genus *Burkholderia.* For example, in live *P*. *aeruginosa* the compound for use according to the present invention is preferably able to lead to a global and complete inhibition of quinolone biosynthesis at low concentrations, such as e.g. between 10 µM and 100 µM.

If not stated otherwise, such as for example partially for the residue R³, the following definitions apply to the terms "halogen", "alkyl group", "cycloalkyl group", "alkenyl group", "cycloalkenyl group", "alkynyl group", "aryl group", and "heteroaryl group". Herein the term "halogen" refers particularly to fluorine atoms, chlorine atoms, bromine atoms, and iodine atoms, preferably bromine atoms and chlorine atoms, most preferably chlorine atoms. The term "alkyl group" refers particularly to a branched or linear alkyl group having 1 to 20, preferably 1 to 12, more preferably 1 to 6, and most preferably 1 to 4 carbon atoms, which can be substituted or unsubstituted. Examples of alkyl groups represent methyl groups, ethyl groups, propyl groups, isopropyl groups, butyl groups, isobutyl groups, tert-butyl groups, pentyl groups, hexyl groups, and heptyl groups. The term "cycloalkyl group" refers particularly to a cycloalkyl group having 3 to 10, preferably 4 to 8, more preferably 5 or 6, and most preferably 6 carbon atoms, which can be substituted or unsubstituted. Examples of cycloalkyl groups represent cyclobutyl groups, cyclopentyl groups, and cyclohexyl groups. The term "alkenyl group" refers particularly to a branched or linear alkenyl group having 2 to 20, preferably 2 to 12, more preferably 2 to 6, and most preferably 2 to 4 carbon atoms, which can be substituted or unsubstituted. Examples of alkenyl groups represent vinyl groups, allyl groups, and crotyl groups. The term "cycloalkenyl group" refers particularly to a cycloalkenyl group having 4 to 10, preferably 5 to 8, more preferably 5 or 6, and most preferably 6 carbon atoms, which can be substituted or unsubstituted. Examples of cycloalkenyl groups represent cyclopentenyl groups, cyclopentadienyl groups, cyclohexyl groups, and cyclohexadienyl groups. The term "alkynyl group" refers particularly to a branched or linear alkynyl group having 2 to 20, preferably 2 to 12, more preferably 2 to 6, and most preferably 2 to 4 carbon atoms, which can be substituted or unsubstituted. Examples of alkynyl groups represent ethynyl groups, 1-propynyl groups, and propargyl groups. The term "aryl group" refers particularly to an aryl group consisting of 1 to 6, preferably 1 to 4, more preferably 1 to 3 aromatic rings, and most preferably 1 ring, which can be substituted or unsubstituted. Examples of aryl groups represent phenyl groups, anthracenyl or naphthyl groups. The term "heteroaryl group" refers particularly to a heteroaryl group consisting of 1 to 6, preferably 1 to 4, more preferably 1 to 3 aromatic rings including heteroatoms, which can be substituted or unsubstituted. Heteroatoms, which are present in heteroaryl groups are for example N, O and S. Examples of heteroaryl groups represent pyridyl groups, pyrimidinyl groups, thienyl groups, furyl groups or pyrrolyl groups.

According to the present invention, the alkyl groups, the cycloalkyl groups, the alkenyl groups, the cycloalkenyl groups, the alkynyl groups, the aryl groups and the heteroaryl groups may be substituted or unsubstituted. The potential substituents are not specifically limited. Accordingly, instead of hydrogen atoms any substituent known in the prior art can be bonded to the further positions of the corresponding groups. For example, the potential substituents may be selected from the group consisting of a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms, a halogen atom, -NL¹L², -NO₂, -CN, -OL³, -SL⁴, -C(O)L⁵, -C(O)NL⁶L⁷, -COOL⁸, and -SO₃L⁹, wherein L¹ to L⁹ are each independently selected from a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms. Accordingly, examples of substituted alkyl groups are aralkyl groups or alkyl groups substituted with e.g. halogen atoms, such as e.g. a trifluoromethyl group or a trichloromethyl group, or any other of the above-mentioned substituents. The term "aralkyl group" refers particularly to an alkyl group wherein one or more hydrogen atoms, preferably terminal hydrogen atoms of the alkyl chain, are replaced by aryl or heteroaryl groups. Examples of aralkyl groups represent benzyl groups or 1- or 2-phenylethyl groups. Preferably, the potential substituents are selected from the group consisting of a branched or linear alkyl group having 1 to 6 carbon atoms, a branched or linear alkenyl group having 2 to 6 carbon atoms, a branched or linear alkynyl group having 2 to 6 carbon atoms, a halogen atom, -NH₂, - NHCH₃, -N(CH₃)₂, -NO₂, -OH, -OCH₃, -OEt, -C(O)H, -C(O)CH₃, -C(O)Et, and -COOH. Moreover, one or more tetravalent carbon atoms (together with the hydrogen atoms bonded thereto), when present, in each of the alkyl groups, the cycloalkyl groups, the alkenyl groups, the cycloalkenyl groups, and the alkynyl groups may each independently be substituted by a member selected from the group consisting of O, (OCH₂CH₂)ₙO, S, (SCH₂CH₂)ₘS, C(O), C(O)O, NR⁸, and C(O)NR⁹, preferably O, (OCH₂CH₂)ₙO, C(O)O, and C(O)NR⁹, wherein n and m are each independently an integer from 1 to 6. Accordingly, for example an alkyl group may be interrupted by e.g. one or more PEG linkers and/or amide or ester bonds. The way the groups are introduced instead of a carbon atom is not specifically limited. For example, a carbon atom may be substituted by C(O)O in the sense of -C(O)O- or -OC(O)- and by C(O)NR⁹ in the sense of -C(O)NR⁹- or -NR⁹C(O)-. According to the present invention, R⁸ and R⁹ are each independently selected from the group consisting of a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, a heteroaryl group having 1 to 3 aromatic rings including heteroatoms, -OG¹, -C(O)G², -C(O)NG³G⁴, -COOG⁵, and -SO₂G⁶. In a preferred embodiment, R⁸ and R⁹ are each independently selected from the group consisting of a hydrogen atom, a branched or linear alkyl group having 1 to 6 carbon atoms, an aryl group having 1 to 3 aromatic rings, -C(O)G², and -SO₂G⁶. Most preferably, R⁸ and R⁹ are each independently selected from the group consisting of a hydrogen atom and a branched or linear alkyl group having 1 to 6 carbon atoms. According to the present invention, G¹ to G⁶ are each independently selected from the group consisting of a hydrogen atom, a branched or linear alkyl group having 1 to 6 carbon atoms, a cycloalkyl group having 4 to 8 carbon atoms, a branched or linear alkenyl group having 2 to 6 carbon atoms, a cycloalkenyl group having 4 to 8 carbon atoms, a branched or linear alkynyl group having 2 to 6 carbon atoms, an aryl group having 1 to 3 aromatic rings, and a heteroaryl group having 1 to 3 aromatic rings including heteroatoms. In a preferred embodiment, G¹ to G⁶ are each independently selected from the group consisting of a hydrogen atom, a branched or linear alkyl group having 1 to 6 carbon atoms, and an aryl group having 1 to 3 aromatic rings.

Most preferably, the alkyl groups, the cycloalkyl groups, the alkenyl groups, the cycloalkenyl groups, the alkynyl groups, the aryl groups, and the heteroaryl groups are unsubstituted. Moreover, in a preferred embodiment the alkyl groups, the alkenyl groups, and the alkynyl groups are linear.

According to the present invention, selected groups may bind to each other to form one or more rings. The corresponding rings may be saturated or unsaturated rings. Furthermore, the rings may contain heteroatoms or may not contain heteroatoms. The sizes of the rings are not particularly limited. For example, the rings may independently be 4- to 8-membered, preferably 5- or 6-membered rings.

The compound according to the present invention may be the compound represented by the general Formula (1) as described above or a pharmaceutically acceptable salt thereof. In case the compound of the present invention is a pharmaceutically acceptable salt of the compound according to general Formula (1), the salt can be formed with inorganic or organic acids or bases. Examples of pharmaceutically acceptable salts comprise, without limitation, non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfonate derived from p-toluenesulfonic acid, sodium salts, potassium salts, magnesium salts, calcium salts, iron salts, zinc salts, aluminum salts, ammonium salts, and others. Such salts can be readily produced by methods known to a person skilled in the art.

Other salts like oxalate derived from oxalic acid, which is not considered as pharmaceutically acceptable, can be appropriately used as intermediates for the production of the compound of the general Formula (1) or a pharmaceutically acceptable salt thereof or physiologically functional derivative or a stereoisomer thereof.

According to the present invention, X is a halogen atom, preferably a fluorine, chlorine, or bromine atom. More preferably, X is a bromine or chlorine atom. Most preferably, X is a chlorine atom.

According to the present invention, Y is NHR³, a hydrogen atom, or a halogen atom, preferably NHR³, a hydrogen atom, or a fluorine atom. Most preferably, Y is NHR³, since it can facilitate accumulation of the compound for use according to the present invention in bacteria, in particular Gram-negative bacteria.

According to the present invention, R¹ and R² are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group. Preferably, R¹ and R² are each independently selected from the group consisting of a hydrogen atom, a substituted or unsubstituted alkyl group, a substituted or unsubstituted alkenyl group, a substituted or unsubstituted alkynyl group, a substituted or unsubstituted aryl group, and a substituted or unsubstituted heteroaryl group. More preferably, R¹ and R² are each independently selected from the group consisting of a hydrogen atom, a substituted or unsubstituted alkyl group, and a substituted or unsubstituted alkenyl group. Most preferably, each of R¹ and R² is hydrogen.

According to the present invention, R³, when present, is selected from the group consisting of a hydrogen atom, a substituted or unsubstituted alkyl group having from 1 to 11, preferably 1 to 8, more preferably 1 to 6, most preferably 1 to 3 carbon atoms, a substituted or unsubstituted cycloalkyl group having from 4 to 12, preferably 4 to 8, most preferably 5 or 6 carbon atoms, a substituted or unsubstituted alkenyl group having from 2 to 11, preferably 2 to 8, more preferably 2 to 6, most preferably 2 or 3 carbon atoms, a substituted or unsubstituted cycloalkenyl group having from 4 to 12, preferably 4 to 8, most preferably 5 or 6 carbon atoms, a substituted or unsubstituted alkynyl group having from 2 to 11, preferably 2 to 8, more preferably 2 to 6, most preferably 2 or 3 carbon atoms, a substituted or unsubstituted aryl group, and a substituted or unsubstituted heteroaryl group. In a preferred embodiment, R³ is selected from the group consisting of a hydrogen atom, a substituted or unsubstituted alkyl group having from 1 to 11, preferably 1 to 8, more preferably 1 to 6, most preferably 1 to 3 carbon atoms, and a substituted or unsubstituted alkenyl group having from 2 to 11, preferably 2 to 8, more preferably 2 to 6, most preferably 2 or 3 carbon atoms. R³ may bind to R⁷ to form a ring, preferably a saturated or unsaturated 5- or 6-membered ring, e.g. a pyrrole ring. Most preferably, R³ is a hydrogen atom.

According to the present invention, R⁴ to R⁷ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, a halogen atom, -NE¹E², -NO₂, -CN, -OE³, -SE⁴, -C(O)E⁵, -C(O)NE⁶E⁷, -COOE⁸, and -SO₃E⁹. E¹ to E⁹ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, preferably E¹ to E⁹ are each independently selected from the group consisting of a hydrogen atom, a substituted or unsubstituted alkyl group, and a substituted or unsubstituted aryl group, most preferably E¹ to E⁹ are each independently selected from the group consisting of a hydrogen atom and a substituted or unsubstituted alkyl group. Preferably, R⁴ to R⁷ are each independently selected from the group consisting of a hydrogen atom, a substituted or unsubstituted alkyl group, a substituted or unsubstituted alkenyl group, a substituted or unsubstituted alkynyl group, a halogen atom, -NE¹E², -NO₂, -OE³, -C(O)E⁵, and -COOE⁸. More preferably, R⁴ to R⁷ are each independently selected from the group consisting of a hydrogen atom, a substituted or unsubstituted alkyl group, a substituted or unsubstituted alkenyl group, -OE³, and a halogen atom. Two or more of R⁴ to R⁷, such as R⁴ and R⁵, R⁵ and R⁶, and/or R⁶ and R⁷, may bind to each other to form one or more rings. Preferably, the rings are saturated or unsaturated, 5- or 6-membered rings, e.g. a pyrrole ring, a 1,6-dioxane ring, or a benzene ring. Most preferably, each of R⁴ to R⁷ is a hydrogen atom.

In one preferred embodiment of the present invention, Y is NHR³ and R³ is H (i.e. Y is NH₂). In this embodiment, it is particularly preferred that X is a bromine or a chlorine atom, most preferably a chlorine atom. Preferably, R¹ and R² are each independently selected from the group consisting of a hydrogen atom, a substituted or unsubstituted alkyl group, and a substituted or unsubstituted alkenyl group, and more preferably each of R¹ and R² is a hydrogen atom. Furthermore, it is preferred in this embodiment that R⁴ to R⁷ are each independently selected from the group consisting of a hydrogen atom, a substituted or unsubstituted alkyl group, a substituted or unsubstituted alkenyl group, -OE³, and a halogen atom, wherein two or more of R⁴ to R⁷ may bind to each other to form one or more rings. In this embodiment, as examples, the present invention more preferably relates to a compound selected from the group consisting of the compound represented by the Formulas (2) to (4), more preferably to a compound selected from the group consisting of the compound represented by the Formulas (2) and (3), most preferably to the compound represented by the Formula (2), or a pharmaceutically acceptable salt thereof.

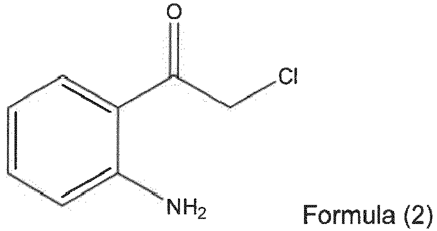

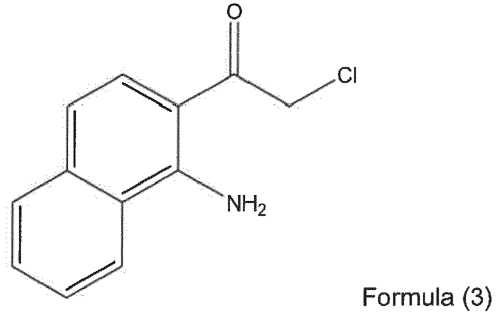

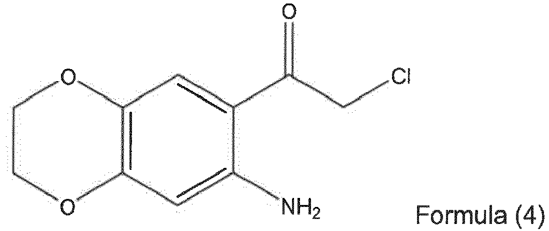

In another preferred embodiment of the present invention, Y is NHR³ and R³ is selected from the group consisting of a substituted or unsubstituted alkyl group having from 1 to 11, preferably 1 to 8, more preferably 1 to 6, most preferably 1 to 3 carbon atoms, and a substituted or unsubstituted alkenyl group having from 2 to 11, preferably 2 to 8, more preferably 2 to 6, most preferably 2 or 3 carbon atoms. Furthermore, R³ binds to R⁷ to form a ring, preferably a saturated or unsaturated 5- or 6-membered ring, most preferably a pyrrole ring (as e.g. shown in Formula (5)). In this embodiment, it is particularly preferred that X is a bromine or a chlorine atom, most preferably a chlorine atom. Preferably, R¹ and R² are each independently selected from the group consisting of a hydrogen atom, a substituted or unsubstituted alkyl group, and a substituted or unsubstituted alkenyl group, and more preferably each of R¹ and R² is a hydrogen atom. Furthermore, it is preferred in this embodiment that R⁴ to R⁷ are each independently selected from the group consisting of a hydrogen atom, a substituted or unsubstituted alkyl group, a substituted or unsubstituted alkenyl group, -OE³, and a halogen atom, wherein two or more of R⁴ to R⁷ may bind to each other to form one or more rings, most preferably each of R⁴ to R⁶ is a hydrogen atom. In this embodiment, as an example, the present invention more preferably relates to the compound represented by the Formula (5), or a pharmaceutically acceptable salt thereof.

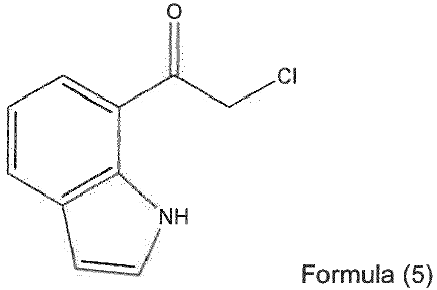

In a further preferred embodiment of the present invention, Y is a halogen atom, preferably a fluorine atom. In this embodiment, it is particularly preferred that X is a bromine or a chlorine atom, most preferably a chlorine atom. Preferably, R¹ and R² are each independently selected from the group consisting of a hydrogen atom, a substituted or unsubstituted alkyl group, and a substituted or unsubstituted alkenyl group, and more preferably each of R¹ and R² is a hydrogen atom. Furthermore, it is preferred in this embodiment that R⁴ to R⁷ are each independently selected from the group consisting of a hydrogen atom, a substituted or unsubstituted alkyl group, a substituted or unsubstituted alkenyl group, -OE³, and a halogen atom, wherein the halogen atom is preferably a fluorine atom, and wherein two or more of R⁴ to R⁷ may bind to each other to form one or more rings. In this embodiment, as an example, the present invention more preferably relates to the compound represented by the Formula (6), or a pharmaceutically acceptable salt thereof. 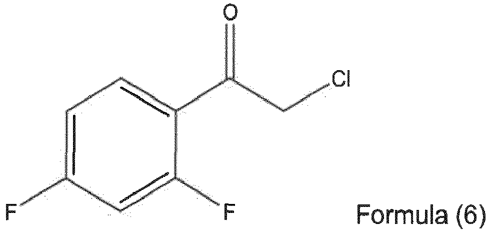 In a further preferred embodiment of the present invention, Y is a hydrogen atom. In this embodiment, it is particularly preferred that X is a bromine or a chlorine atom, most preferably a chlorine atom. Preferably, R¹ and R² are each independently selected from the group consisting of a hydrogen atom, a substituted or unsubstituted alkyl group, and a substituted or unsubstituted alkenyl group, and more preferably each of R¹ and R² is a hydrogen atom. Furthermore, it is preferred in this embodiment that R⁴ to R⁷ are each independently selected from the group consisting of a hydrogen atom, a substituted or unsubstituted alkyl group, a substituted or unsubstituted alkenyl group, -OE³, and a halogen atom, wherein two or more of R⁴ to R⁷ may bind to each other to form one or more rings. Most preferably, each of R⁴ to R⁷ is a hydrogen atom. In this embodiment, as an example, the present invention more preferably relates to the compound represented by the Formula (7), or a pharmaceutically acceptable salt thereof.

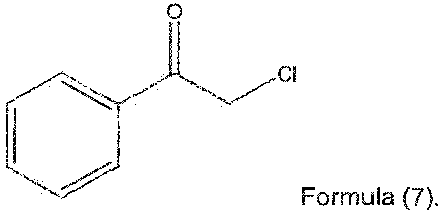

The above embodiments can be combined with each other without any particular limitation. The above statements and definitions given with respect to the specific embodiments analogously apply to each respective embodiment when combined with the other embodiments. In this embodiment, as examples, the present invention more preferably relates to a compound selected from the group consisting of the compounds represented by the Formulas (2) to (7), more preferably to the compounds represented by the Formulas (2), (3), and (5), most preferably to the compounds represented by the Formulas (2) and (5), or pharmaceutically acceptable salts thereof.

The pathogenic bacteria, which can be treated with the compound for use according to the present invention, are preferably pathogenic bacteria mediating pathogenicity by quinolone dependent production of virulence factors, such as e.g. *Pseudomonas aeruginosa* and species of the genus *Burkholderia.* For example, the *Pseudomonas aeruginosa* may include different strains such as *Pseudomonas aeruginosa* PAO1 and *Pseudomonas aeruginosa* PA14 as well as clinical strains of *Pseudomonas aeruginosa.* Species of the genus *Burkholderia* may be selected from the *Burkholderia cepacia* complex such as for example *Burkholderia cepacia, Burkholderia cenocepacia, Burkholderia amibfaria,* or *Burkholderia multivorans* or may include other species of *Burkholderia* such as *Burkholderia mallei* and *Burkholderia pseudomallei.*

In a further preferred embodiment, the treatable bacteria are multi-resistant bacteria, more preferably multi-resistant *Pseudomonas aeruginosa* and species of the genus *Burkholderia,* more preferably multi-resistant *Pseudomonas aeruginosa.* Examples of multi-resistant *Pseudomonas aeruginosa* strains are represented by, but are not limited to vancomycin resistant, fluoroquinolone resistant, aminoglycoside resistant, carbapenem resistant and extended-spectrum β-lactamases (ESBL) positive strains of *Pseudomonas aeruginosa.* Furthermore, examples of multi-resistant species of the genus *Burkholderia* are represented by, but are not limited to members of the *Burkholderia cepacia* complex such as for example *Burkholderia cepacia, Burkholderia cenocepacia, Burkholderia amibfaria,* and *Burkholderia multivorans* or other species of *Burkholderia* such as *Burkholderia mallei* and *Burkholderia pseudomallei.*

The conditions and infections, which can be treated with the compound for use according to the present invention are for example caused by pathogenic bacteria mediating pathogenicity by quinolone dependent production of virulence factors, such as e.g. *Pseudomonas aeruginosa* and species of the genus *Burkholderia.* For example, such conditions and infections include, but are not limited to, sepsis, wound infections, endocarditis, meningitis, and chronic respiratory infections in cystic fibrosis patients.

In a preferred embodiment, the compound for use according to the present invention has a IC₅₀ value for inhibition of quinolone biosynthesis, preferably inhibition of PqsD and HmqD, more preferably inhibition of PqsD, of 10 µM or less, more preferably 5.0 µM or less, more preferably 3.0 µM or less, more preferably 2.6 µM or less, more preferably 2.2 µM or less, most preferably 2.0 µM or less. The lower limit of the IC₅₀ value is generally not specifically limited. For example, the lower limit of the IC₅₀ value may be 0.001 nM.

According to the present invention, the compound for use according to the present application may be administered by any administration route known in the art being suitable for delivering a medicament to a vertebrate, such as a mammal. The route of administration does not exhibit particular limitations and includes for example oral application, topic application, intravenous application and intraperitoneal application. The compound also may be administered topically as ointment, by powders, drops or transdermal patch, or as an oral or nasal spray.

The dosage of the compound for use according to the present application can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 µg/kg/day to 100 mg/kg/day animal body weight preferably 5 µg/kg/day to 50 mg/kg/day. Suitable dosage rates for larger mammals, for example humans, are of the order of from about 1 mg to 4 g/day, conveniently administered once, in divided doses such as e.g. 2 to 4 times a day, or in sustained release form. Moreover, the compound for use according to the present application can be applied topically to a locally defined site of infection, including but not limited to the eye, lung, or skin. In these cases, different dosages may be applied directly to the site of infection ranging from 1 ng/application to 5 g/application, preferably 1 ng/application to 1 g/application, more preferably 1 ng/application to 100 mg/application. Applications may vary from a single dose application or one application per day or one application every second day, to several applications per day such as two, three, four or five applications/day.

In another aspect, the present invention relates to a pharmaceutical composition comprising the compound according to the present invention as defined above in a pharmaceutically active amount, and optionally a pharmaceutically acceptable carrier, excipient or diluent. Preferably, the pharmaceutical composition for use in the treatment of infections with pathogenic bacteria. The above statements and definitions analogously apply to this aspect of the present invention. The compound of the present invention can be administered *per se* or in the form of pharmaceutical preparations.

The term "medicament" as used herein relates to any pharmaceutical composition comprising at least the compound according to the present invention in a pharmaceutically active amount.

The concentration of the compound of the present invention in the pharmaceutical composition of the present invention is not particularly limited. Preferably, the concentration of the compound of the present invention in the pharmaceutical composition is from 0.1 µM to 5 M, more preferably from 5 µM to 5 M, and most preferably from 10 µM to 100 mM.

The figures show:
Figure 1: Biosynthesis of the different quinolone classes of *Pseudomonas aeruginosa* and their biological effects. 2-HABA: 2'-hydoxyaminobenzoylacetate
Figure 2: Scheme of the competitive labelling platform.
Figure 3: Live *E. coli* BL21 cells overexpressing PqsD labelled by probe CA (100 µM) resulted in a strong band for PqsD compared to the parent BL21 strain.
Figure 4: Dose-down of probe CA with live *E. coli* overexpressing PqsD.
Figure 5: Mass spectrum of the modified peptide fragment after *in situ* labelling of *E. coli* BL21 cells overexpressing PqsD by probe CA. C* = modified active site cysteine (Cys112) of PqsD.
Figures 6 to 11: Concentration-dependent inhibition curves of compounds represented by the Formulas (2) to (7) against their respective overexpressed target PqsD in live *E. coli* BL21 cells. Calculated IC₅₀ values correspond to means ± SD for three replicates.
Figure 12: Tryptic peptide fragment with compound represented by the Formula (2) covalently bound to the active site cysteine of PqsD after *in situ* inhibition.
Figure 13: Growth curves of *Pseudomonas aeruginosa* PAO1 and PA14 in presence of different concentrations of compounds of Formulas (2) and (5). All experiments were performed in 3 biological replicates.
Figures 14 to 16: Structures and fragmentation of AQs (Figure 14), AQNOs (Figure 15), as well as PQS and phenazines (Figure 16).
Figure 17: Quinolone and phenazine inhibition in *P*. *aeruginosa* PAO1 cultures treated with the compound represented by Formula (2). a) Structures of quinolones and phenazines analyzed by LC-MS/MS. b) Chromatograms of mass transitions from extracted supernatants after incubation with of Formula (2) at different concentrations for 24 h. c) Percentage of inhibition of extracellular quinolones and phenazines.
Figure 18: Integrated area of monitored mass transitions of AQs and AQNOs of *Pseudomonas aeruginosa* PAO1 after different incubation times at 37 °C. Experiments were performed in 3 biological replicates.
Figure 19: Inhibition of AQ, AQNO and phenazine production of *Pseudomonas aeruginosa* PA14 after treatment with inhibitor of Formula (2) (10, 50 and 100 µM) for 24 h at 37 °C was analyzed by LC-MS/MS in SRM mode. Chromatograms (a) and percentage inhibition (b) of the product ions of all monitored mass transitions in SRM scan mode. Experiments were performed in 4 biological replicates.
Figure 20: Area of monitored mass transitions of AQs, AQNOs and phenazines after treatment of *Pseudomonas aeruginosa* PAO1 (a) or PA14 (b) with compound of Formula (2) in final concentrations of 10, 50 and 100 µM. Experiments were performed in 4 biological replicates.
Figure 21: Inhibition of AQ, AQNO and phenazine production of *P. aeruginosa* PAO1 (a) and PA14 (b) after treatment with compound Formula (5) at 100 µM for 27 h at 37 °C was analyzed by LC-MS/MS in SRM mode. Percentage of inhibition is shown for the product ions of all monitored mass transitions in SRM scan mode. Experiments were performed in 3 biological replicates.
Figure 22: HHQ production of *P. aeruginosa* PAO1 inhibited by treatment with the compound represented by Formula (2) (50 µM) for 11 h at 37 °C was restored by 2-ABA. Percentage of relative HHQ production is shown for the product ions of all monitored mass transitions in SRM scan mode. Experiments were performed in 3 biological replicates.

The present invention will be further illustrated in the following examples without being limited thereto.

### Experimental procedures:

### General

All solvents and chemical reagents for synthesis and LC-MS/MS analysis were purchased from Sigma-Aldrich, VWR or Carl Roth. The LC-MS standard phenazine-1-carboxamide was purchased from ChemCruz, 1-phenazinol from TCI and pyocyanin from Sigma-Aldrich. The compounds represented by the Formulas (3) to (7) were obtained from commercial sources. Flash chromatography was performed using silica gel. NMR spectra were obtained using a Bruker Avance III 400 NMR instrument equipped with a BBFO plus probe and calibrated on the residual solvent peak. Chemical shifts are reported in ppm and coupling constants (*J*) are given in Hz. High-resolution mass spectra were recorded by an ESI-TOF MS (Bruker Daltonics amicroTOFII) equipped with a Chromolith FastGaradient Rp18e 50*2 mm column (Merck). Low-resolution mass analysis was performed using an ESI-IT MS (Bruker Daltonics Esquire 3000plus) with a Nucleoshell 50*2 mm RP-18 2.7 µm column (Macherey-Nagel). For the SDS-gel preparation and SDS-PAGE in general the PeqLab System was used. The recording and analysis of the gels was performed with the Fusion-FX7 Advanced of Vilber Lourmat (Eberhardzell, Germany) using the software CaptAdvance.

For preparation of overnight cultures used in cellular assays, a small amount of a bacterial cryo-stock (15% glycerol, stored at -80 °C) was inoculated in 5 mL LB medium in sterile 13 mL polypropylene tubes (Sarstedt, ref 62.515.028), supplemented with antibiotics if indicated, and grown for 14-16 h at 37 °C (180 rpm).

### Click Chemistry

Click chemistry is performed using 19.5 µL cell lysate, 1 µL of a 21.5 % SDS solution, 1 µL of a 0.325 mM rhodamine azide stock in DMSO, and 1.5 µL of a 2 mM tris(3-hydroxypropyltriazolylmethyl)amine stock in *tert*-butanol/DMSO (8:2 v/v). To start the cycloaddition reaction, 1 µL of a freshly prepared 25 mM tris(2-carboxyethyl)phosphine hydrochloride solution in water and 1 µL of a 25 mM CuSO₄ stock solution in water are added. The samples are incubated for 1 h at room temperature and quenched by addition of 2 x SDS loading buffer (63 mM Tris-HCI, 10% (v/v) Glycerol, 2% (w/v) SDS, 0.0025% (w/v) Bromophenol blue, 10% (v/v) β-mercaptoethanol; dissolved in water).

### Synthesis Example

Synthesis of 2'-amino-1-chloro-acetophenone (Compound represented by the Formula (2))

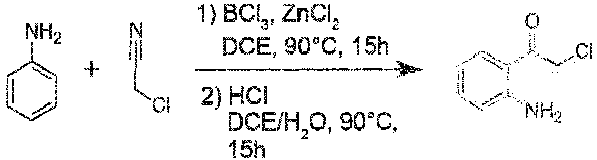

In accordance to the literature (Carrie A. B. Wagner, S. A. M., Two robust, efficient syntheses of [phenyl ring-U-14C]indole through use of [phenyl ring-U-14C]aniline. Labelled Compounds and Radiopharmaceuticals 2006, 49 (7), 615-622), ZnCl₂ (4.4 g, 32 mmol, 4.0 eq.) was dried under high vacuum, and under nitrogen atmosphere dry 1,2-dichloroethane (24.0 mL) was added. Aniline (0.75 mL, 8 mmol, 1.0 eq.) and chloroacetonitrile (1.75 mL, 28 mmol, 3.4 eq.) were added and BCl₃ (1 M in dichloromethane (DCM), 24 mL, 3.0 eq.) was added slowly. The mixture was heated to reflux (90°C, oil bath) for 15 h. It was cooled to room temperature, 1 M aq. HCl (24 mL) was added and heated to reflux (100°C, oil bath) for 90 min. It was cooled to room temperature, DCM (200 mL) and water (200 mL) were added and the aqueous layer was extracted with DCM (3 x 100 mL). The organic layers were combined, dried over MgSO₄ and evaporated to give a brown solid (745 mg). Flash column chromatography on silica gel was performed in petroleum ether/ethyl acetate (PE/EA) 7:1 to yield pure product as yellow solid (235 mg, 1.4 mmol, 17%).

Rf: 0.62 (PE/EA 5:1); ¹H NMR (400 MHz, C₆D₆): *δ*=7.07-7.05 (m, 1H, 6'C*H*), 6.94-6.90 (m, 1H, 4'C*H*), 6.33-6.29 (m, 1H, 5'C*H*), 6.07-6.05 (m, 1H, 3'C*H*), 5.84 (s, 2H, N*H*₂), 3.94 (s, 2H, C*H₂*Cl); ¹³C NMR (101 MHz, C₆D₆) *δ*=192.5 (*C*=O), 151.5 (2'*C*), 134.8 (4'*C*), 131.8 (6'*C*), 117.4 (3'*C*), 115.5 (5'*C*), 115.4 (1'*C*), 46.2 (*C*H₂Cl); HRMS: m/z measured= 170.0373 [M+H]⁺, m/z calculated= 170.0371 [M+H]⁺, Δppm = 1.2.

### Example 1: Live-cell labelling strategy for determining PqsD activity in E.coli overexpressing PqsD

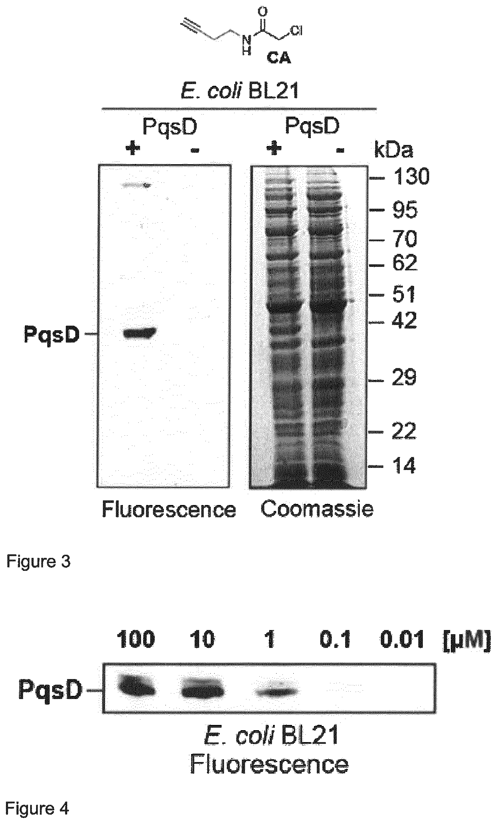

A live-cell labelling strategy (Figure 2) is applied by using an α-chloroacetamide (CA) probe (Figure 3). For this purpose, intact cells are incubated with the probe, excess probe is removed by washing steps and after cell lysis click chemistry is employed to append a fluorescent tetramethylrhodamine tag to the terminal alkyne group for visualization by SDS-polyacrylamide gel electrophoresis (PAGE). *Escherichia coli* cells overexpressing PqsD were used as *in situ* model for in-cell labelling.

*E. coli* BL21 cells containing pDEST/pqsD (wild type PqsD) or pDEST/pqsDmutant (Cys112Ala mutant) (cf. Prothiwa, M.; Szamosvári, D.; Glasmacher, S.; Böttcher, T., Chemical probes for competitive profiling of the quorum sensing signal synthase PqsD of Pseudomonas aeruginosa. Beilstein Journal of Organic Chemistry 2016, 12, 2784-2792) were grown in an overnight culture substituted with 100 µg/mL carbenicillin. On the next day, the culture was inoculated 1:50 in LB media, supplemented with 100 µg/mL carbenicillin and incubated at 37°C (180 rpm) to an OD₆₀₀ of 0.5. Next, 0.2 µg/mL anhydrotetracyclin was added to induce protein expression, and the culture was incubated at 37°C for 120 min (180 rpm). For each sample, 2 mL of induced bacterial culture was transferred into a 2 mL Eppendorf tube, the cells pelleted by centrifugation (4500 rcf, 5 min, 4°C), and the cell pellet washed with 1 mL PBS before re-suspending in 100 µL PBS. 1 µL of compound stock in DMSO or DMSO as vehicle was added for the indicated final concentration, mixed gently and pre-incubated for 60 min at 37 °C in a shaking incubator (180 rpm). Then, 1 µL of CA stock in DMSO was added for a final concentration of 100 µM and incubated for 60 min at 37°C (180 rpm). After incubation, the cells were pelleted by centrifugation (4500 rcf, 5 min, 4 °C), washed (2 x 1 mL PBS), the cell pellet was re-suspended in 100 µL PBS, lysed by ultrasound treatment (10 % amplitude, 0.5 sec on, 1.0 sec off, 20 pulses, Branson Digital Sonifier), and the cell debris removed by centrifugation (12000 g, 10 min, 4 °C). The resulting lysates were used for click chemistry and SDS-PAGE. After fluorescence scanning, Coomassie staining was applied to compare protein concentrations in the gel and thereby validate the experiments.

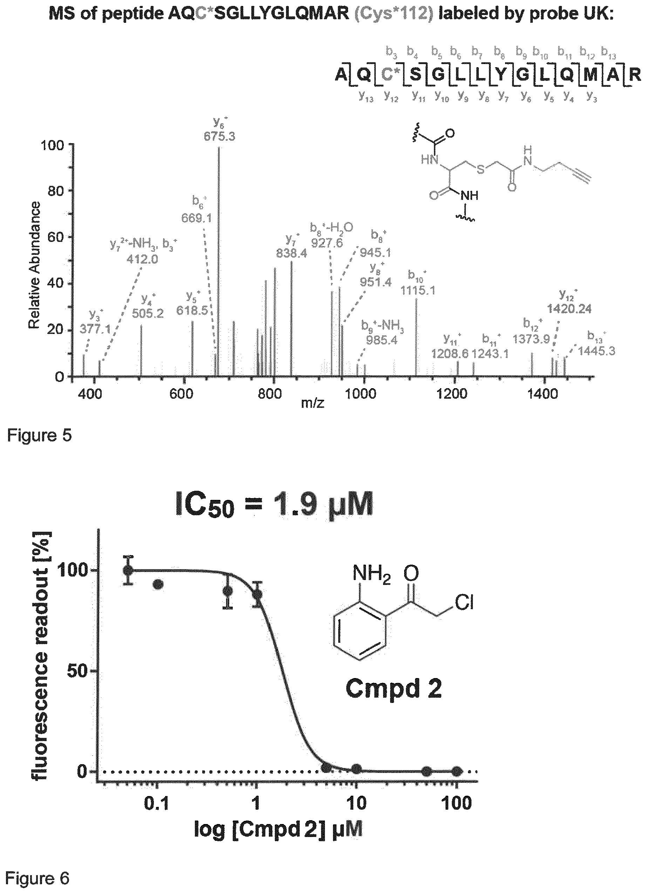

The incubation of cells with 100 µM of CA probe revealed a band only for *E. coli* cells expressing PqsD but not for the parent *E*. *coli* strain (Figure 3). The labelling hereby achieved remarkable sensitivity down to submicromolar probe concentrations (Figure 4). The band of the corresponding protein size was cut out and after tryptic digest subjected to proteomic analysis by mass spectrometry. The results confirmed the sequence of PqsD and in addition the active site cysteine as the site of covalent attachment of the probe (Figure 5).

### Example 2: In situ determination of IC₅₀ values

*E. coli* BL21 cells containing pDEST/pqsD (wild type PqsD) were grown in an overnight culture substituted with 100 µg/mL carbenicillin. On the next day, the culture was inoculated 1:50 in LB medium, supplemented with 100 µg/mL carbenicillin and incubated at 37°C (180 rpm) to an OD₆₀₀ of 0.5. Next, 0.2 µg/mL anhydrotetracyclin was added to induce protein expression, and the culture was incubated at 37°C for 120 min (180 rpm). For each sample, 2 mL of induced bacterial culture was transferred in a 2 mL Eppendorf tube, the cells pelleted by centrifugation (4500 rcf, 5 min, 4 °C), and the cell pellet washed with 1 mL PBS before re-suspending in 100 µL PBS. To this cell suspension, 1 µL of inhibitor stock in DMSO was added at indicated concentrations (n = 3) and incubated for 30 min at 37 °C (180 rpm). Afterwards, the CA probe was added to give a final concentration of 100 µM and incubated 60 min at 37 °C (180 rpm). The cells were washed (2 x 1 mL PBS), lysed by sonication, cell debris was removed by centrifugation and rhodamine attached via click chemistry. Subsequently, the samples were analyzed by SDS-PAGE and in-gel visualization. Integrated fluorescence intensities of the bands were measured using ImageJ software and IC₅₀ values calculated from a dose-response curve generated using GraphPad Prism software.

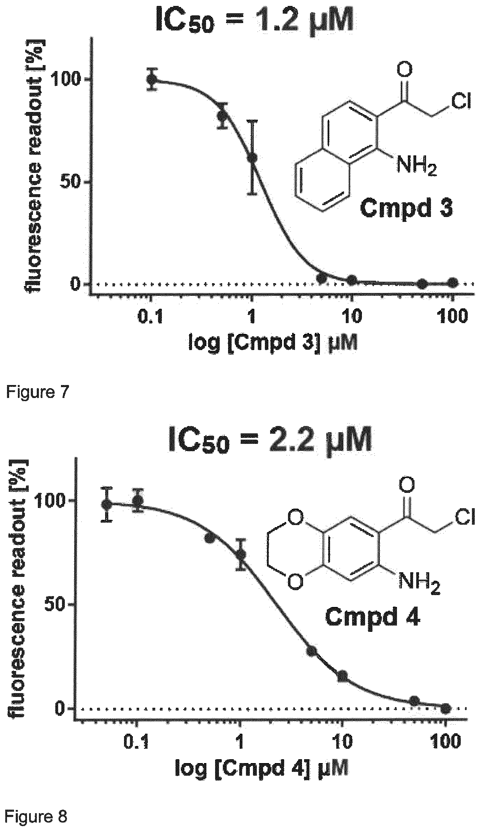

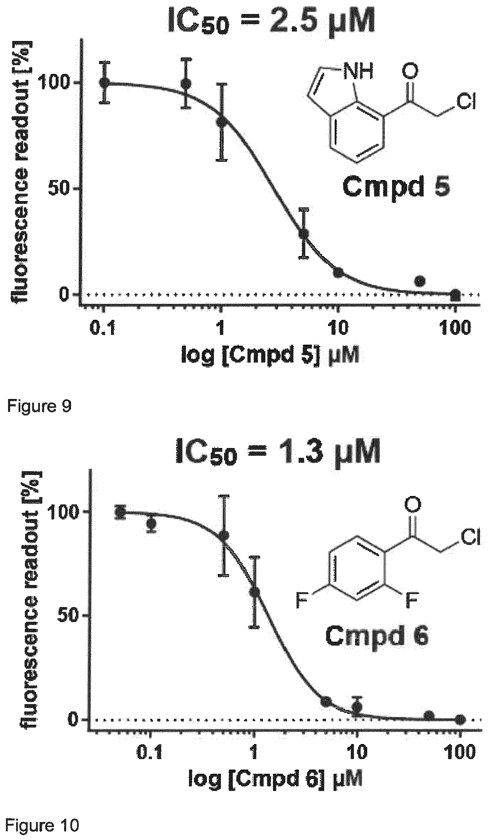

IC₅₀ values for PqsD inhibition in living cells were determined by quantifying the intensity of competitive probe labelling over a broad range of concentrations ranging from 0.05 to 100 µM of the compounds represented by the Formulas (2) to (7) (Figures 6 to 11). Compounds represented by Formulas (2) to (7) with an α-chloroketone motif showed potent inhibition and exhibited IC₅₀ values of 1-3 µM (Figures 6 to 11).

### Example 3: Proteomic analysis of active site modification

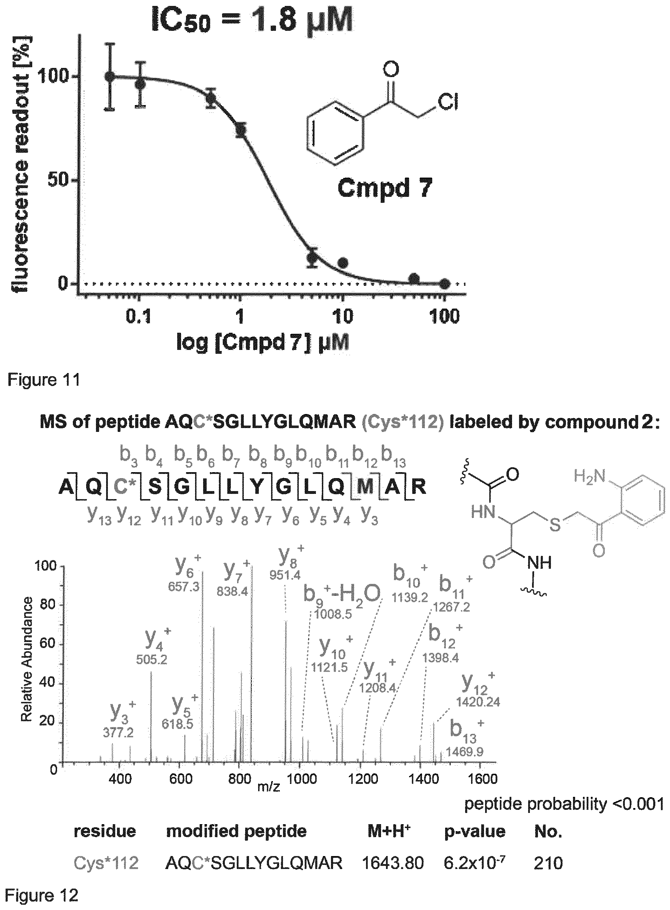

*In situ* labelling was performed, and 2 x SDS loading buffer was added directly to the resulting lysates. After SDS-PAGE and Coomassie staining, the protein band corresponding to PqsD was cut out of the gel. Then, tryptic digestion, sample preparation and LC-MS analysis were performed: Coomassie gel bands were washed in MilliQ for 15 sec, bands cut as close as possible and placed in Eppendorf tubes. For reduction and alkylation, a solution of 10 mM DTT in 50 mM NH₄CO₃ was added and incubated for 60 min at 56 °C. The DTT solution was removed and a solution of 50 mM iodoacetamide in 50 mM NH₄CO₃ was added and incubated for 60 min at room temperature (rt). In a final washing step, the gel pieces were washed with 100 µL MilliQ water and 100 µL 50 mM NH₄CO₃ solution, then the solution was removed, and the gel dehydrated with acetonitrile/MilliQ water (3:2). The washing step was repeated until the gel pieces turned colorless. Next, the dehydration solution was removed, and acetonitrile was added for 10 sec before drying on air until complete dryness. The gel pieces were incubated for 45 sec on ice in a freshly prepared cold buffer containing trypsin (10.0 ng/µL) and 50 mM NH₄CO₃. Afterwards the solution was removed, and the gel incubated overnight in 50 mM NH₄CO₃ solution at 37 °C. On the next day the supernatant was removed, and peptides were extracted by incubation in acetonitrile / 0.1 % TFA in MilliQ water (3:2) for 60 sec at rt. The elution was collected, and the gel incubated in acetonitrile for 15 sec at rt. The combined washes and elutions were dried via SpeedVac and analyzed by an Orbitrap Fusion with EASY-nLC1200 instrument. The mass spectrum of the tryptic peptide fragment with the compound represented by Formula (2) covalently bound to the active site cysteine of PqsD after *in situ* inhibition is shown in Figure 12.

The proteomic analysis of PqsD inhibited by the compound represented by Formula (2) in live cells, confirmed that the compound only bound to the active site cysteine (Cys112) although the protein structure comprises in total six cysteines, five of which are not involved in catalysis (Figure 12). This indicates a highly selective mechanism-based mode of action.

### Example 4: Evaluation of the influence of the compounds represented by Formulas (2) and (5) on the growth of P. aeruginosa

### Determination of Minimum Inhibitory Concentration (MIC value) of the compound represented by Formula (2)

An overnight culture of *P. aeruginosa* PAO1 or PA14 was diluted 1:1000 in LB media. Afterwards, 99 µL of bacterial suspension and 1 µL of a corresponding stock solution of the compound represented by Formula (2) in DMSO was added to each well of a sterile, round-bottom 96-well plate. The compound represented by Formula (2) was applied in final concentrations of 1000, 500, 100, 50, 10, 5, 1, 0.5, 0.1, 0.05 µM in triplicates. The plate was incubated for 24 h at 37 °C (180 rpm). The MIC value was considered as the lowest concentration of compound of Formula (2) that fully inhibits the visible growth. MIC values for compound of Formula (2) were shown to be > 1000 µM against *P. aeruginosa* PAO1 and PA14 after incubation.

### Bacterial growth curve analysis of the compounds represented by Formulas (2) and (5)

In a sterile 15 mL polypropylene centrifuge tubes with screw caps (Roth) 20 µL of a respective DMSO Stock of the compounds represented by Formulas (2) and (5), or 20 µL DMSO as vehicle control was added for 1 % final DMSO concentration. Then, 2 mL of LB medium and 6 µL of an overnight culture of *P. aeruginosa* PA01 or PA14 was added per sample. The samples were incubated for 27 h at 37 °C (190 rpm). At indicated incubation times 50 µL LB medium and 50 µL of culture were transferred in a cuvette, mixed and the adsorption measured at 600 nm.

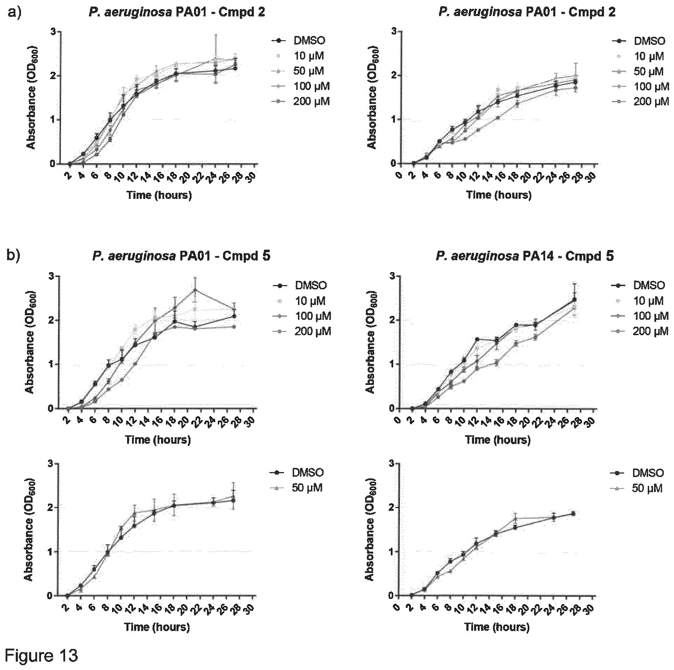

The above experiments showed that growth remained largely unaffected with the compounds represented by Formulas (2) and (5) at concentrations up to 100 µM, ruling out that potential effects on virulence would be an artifact of growth inhibition (Figure 13). Therefore, it was possible to evaluate the effects of the compound for use of the present invention on the biosynthesis of quinolones.

### Example 5: Evaluation of quinolone and phenazine inhibition in P. aeruginosa PAO1 cultures treated with the compound for use according to the present invention

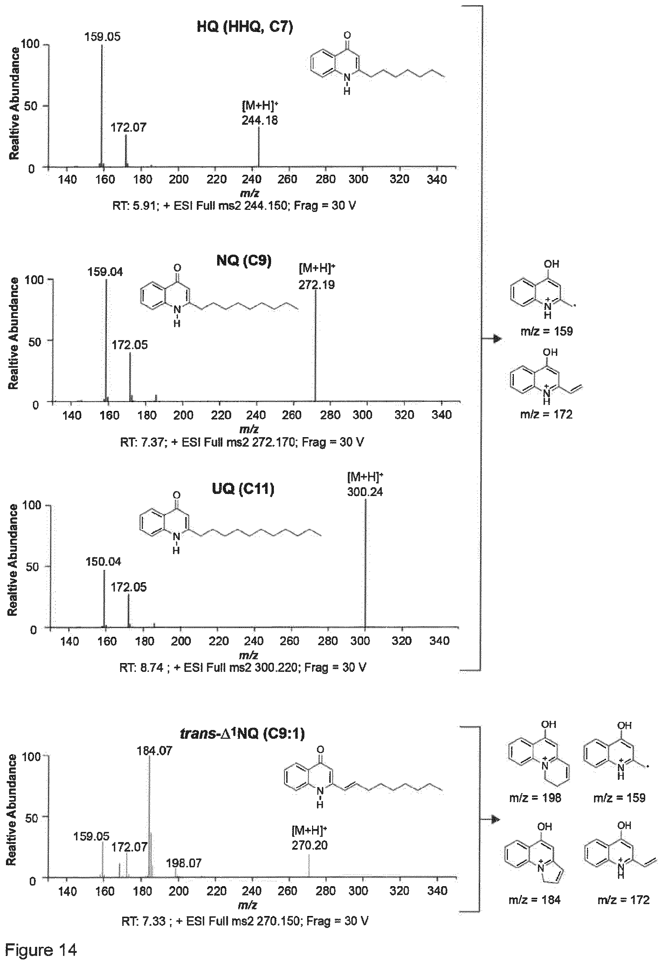

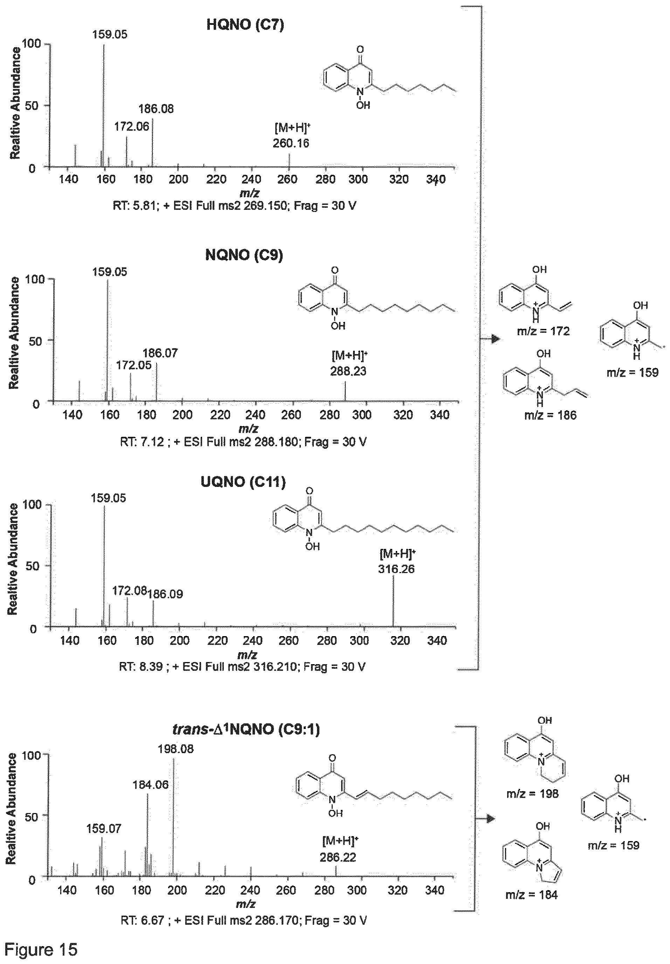

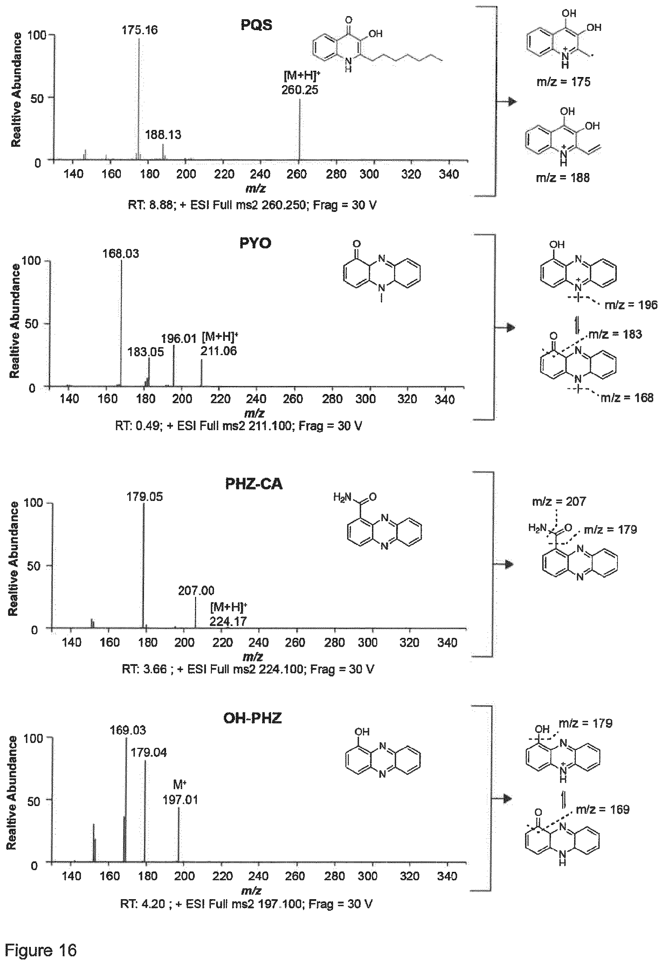

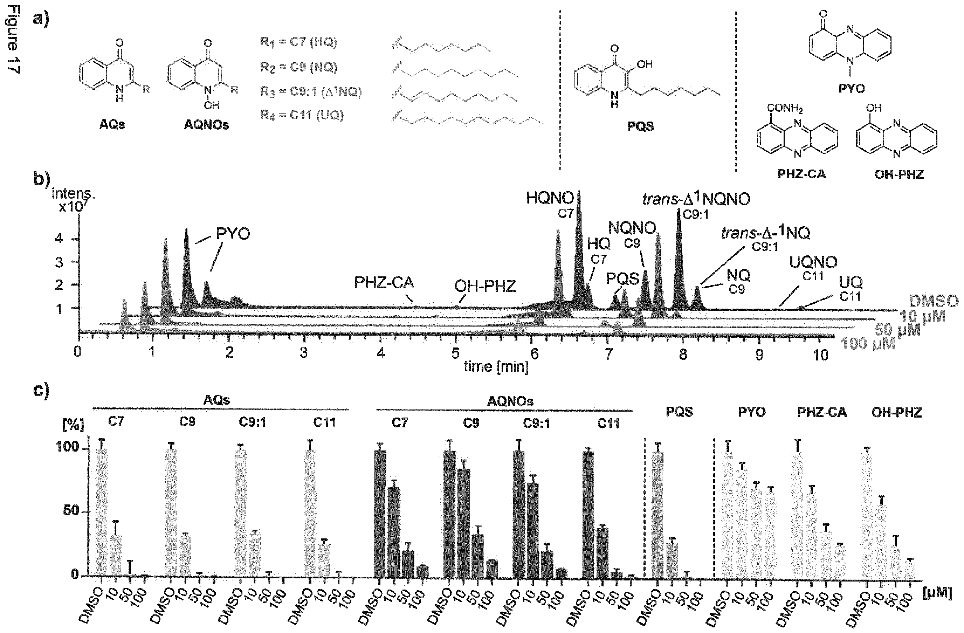

A LC-MS/MS method was established using characteristic mass transitions for quantitative analysis of metabolites in the supernatants of P. *aeruginosa* (Table 1, Figures 14 to 16). A library of phenazines and synthetic AQs and AQNOs served as standards (Szamosvári, D.; Böttcher, T., Angew Chem Int Ed Engl 2017, 56 (25), 7271-7275). There were also analyzed phenazines, including phenazine-1-carboxamide (PHZ-CA), 1-hydroxyphenazine (1-OH-PHZ), and pyocyanin (PYO), which are produced as virulence factors partially under control of the PQS quorum sensing system (Figure 17a).

**Table 1**

| **Retention time [min]** | **Substance** | **Transition [*m*/*z*]** | **Standard equation, R-value** | **Standard concentrations [ng/mL]** |
|---|---|---|---|---|
| 0.61 | **PYO** | 211/168 | log(Y) = 6.53919+0.793204*log(X) R^2 = 0.9955 | 10, 1000, 10000, 100000, 500000 |
| 3.66 | **PHZ-CA** | 224/179 | log(Y) = 3.62878+0.945636*log(X) R^2 = 0.9997 | 1, 10, 100, 10000, 100000 |
| 4.20 | **OH-PHZ** | 197/169 | log(Y) = 2.75459+1.17098*log(X)-0.0223978*(log(X))^2 R^2 = 0.9993 | 390.6, 1562.5, 6250, 25000, 100000 |
| 5.91 | **HQ (HHQ, C7)** | 244/159 | log(Y) = 4.8823+0.930673*log(X) R^2 = 0.9993 | 1, 10, 100, 1000, 10000 |
| 5.81 | **HQNO (C7)** | 160/159 | log(Y) = 3.99339+1.05224*log(X) R^2 = 0.9981 | 1, 10, 100, 1000, 10000 |
| 8.88 | **PQS** | 260/175 | log(Y) = 3.41946+1.27541*log(X)-0.0158104*(log(X))^2 R^2 = 0.9989 | 97.7, 390.6, 1562.5, 6250, 25000 |
| 6.67 | ***trans*-Δ¹-NQNO (C9:1)** | 286/198 | log(Y) = 3.95285+1.02441*log(X) R^2 = 0.9991 | 1, 10, 100, 1000, 10000 |
| 7.33 | ***trans*-Δ¹-NQ (C9:1)** | 270/184 | log(Y) = 4.80012+0.969551*log(X) R^2 = 0.9995 | 1, 10, 100, 1000, 10000 |
| 7.37 | **NQ (C9)** | 272/159 | log(Y) = 4.80343+0.953124*log(X) R^2 = 0.9989 | 1, 10, 100, 1000, 10000 |
| 7.12 | **NQNO (C9)** | 288/159 | log(Y) = 4.4326+0.994275*log(X) R^2 = 0.9981 | 1, 10, 100, 1000, 10000 |
| 8.39 | **UQNO (C11)** | 316/159 | log(Y) = 4.46167+0.994573*log(X) R^2 = 0.9989 | 1, 10, 100, 1000, 10000 |
| 8.74 | **UQ (C11)** | 300/159 | log(Y) = 4.84332+0.96204*log(X) R^2 = 0.9990 | 1, 10, 100, 1000, 10000 |

### LC-MS/MS analysis

Ultra-high performance liquid chromatography was performed on a Dionex Ultimate 3000 UHPLC (Thermo Fisher Scientific, Waltham, MA) using a Nucleodur C18 Gravity-SB 150 x 2 mm, 3 µm column (Macherey-Nagel). The flow rate was 0.5 mL/min and the columns temperature was held at 40 °C. The injection volume was 5 µL using pick up injection mode. Eluent A was 0.1 % formic acid in water and eluent B was acetonitrile. The gradient was 20-100 % B in 10 min, 100 % B for 2 min, 100-20 % B in 1 min and 20 % B for 2 min. MS/MS analysis was performed by a Finnigan™ TSQ® Quantum (Thermo electron corporation) mass spectrometer in the SRM (Selected Reaction Monitoring) scan mode. As ion source a heated-electrospray ionization (HESI-II probe, Thermo scientific) was used. In the optimized conditions the ion spray voltage was 3500 V, vaporizer temperature 300 °C, capillary temperature 380 °C, sheath gas pressure 60 psi, ion sweep gas pressure 2 psi, aux gas 10 psi. The tube lens offset was 80 V and skimmer offset 0. Data were acquired in a mass range of *m*/*z* 130-350, and MS/MS acquisition of all compounds was performed in positive mode and fixed collision energy of 30 eV. Dwell time was 0.050 sec for all compounds.
The software Quan Browser Thermo Xcalibur 3.1.66.10 was used for quantitative analysis. The standard peak area of the product ions was fitted by linear regression versus the known concentrations to generate a standard curve.

### Analysis of AQ and AQNO levels over a 24 h period

10 µL DMSO as vehicle control (0.33 % final DMSO concentration) was added to 3 mL LB medium in 15 mL polypropylene centrifuge tubes with screw caps (VWR). Afterwards 10 µL of a *P*. *aeruginosa* PAO1 or PA14 overnight culture was added. The caps of the sample tubes were slightly opened and fixed in a defined position by tape to ensure equal oxygen delivery to all samples. The samples were incubated for 5, 6, 7, 9, 11 and 24 h at 37 °C (180 rpm). Afterwards, samples were centrifuged, supernatants sterile filtered and AQs and AQNOs extracted and quantified by LC-MS/MS according to the previously described protocol. The experiment was performed in 3 biological replicates.

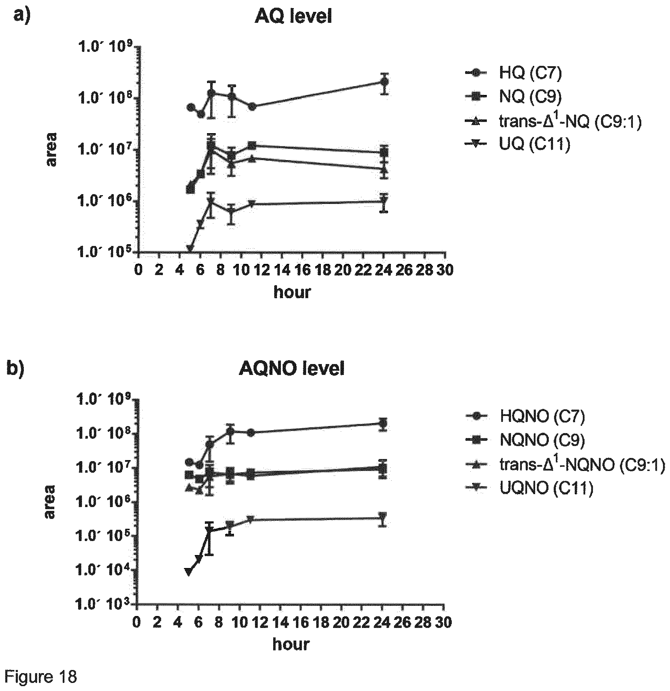

Quantifying quinolone production over time revealed that AQs and AQNOs are produced between 5 and 10 h and then remain constant to 24 h (Figure 18).

### Evaluation of inhibition

Cultures of *P*. *aeruginosa* strains PAO1 and PA14 were incubated with different concentrations of the compound represented by Formula (2) for 24 h and extracted culture supernatants were used for LC-MS/MS analysis.

### Inhibition assay:

10 µL of a respective DMSO stock solution of compound represented by Formula (2) or 10 µL DMSO as vehicle control (0.33 % final DMSO concentration) was added to 3 mL LB medium in 15 mL polypropylene centrifuge tubes with screw caps (VWR) to obtain final concentrations of 10, 50 and 100 µM. Afterwards 10 µL of a *P*. *aeruginosa* PAO1 or PA14 overnight culture was added. The caps of the sample tubes were slightly opened and fixed in a defined position by tape to ensure equal oxygen delivery to all samples. The samples were incubated for 24 h at 37 °C (180 rpm). Afterwards, the samples were centrifuged and the supernatants sterile filtered. The inhibition assay was performed in 3-4 biological replicates.

### Sample preparation:

500 µL of sterile supernatant was transferred in 1.5 mL glass vials, 500 µL chloroform was added and vortexed thoroughly. Then, 200 µL of the organic layer was transferred in a clean 1.5 mL glass vial and the solvent evaporated by a gentle stream of nitrogen. The extract was stored at -80 °C until measurement. For analysis, 50 µL acetonitrile/water (1:1) was added to each sample.

### Preparation of calibration standards:

MeOH stock solutions of PQS, AQs, AQNOs and phenazines were prepared at 1 mg/mL in glass vials and stored at -80 °C. A series of standard solutions was prepared freshly before LC-MS/MS analysis by serial dilutions of the stock solution of each analyte in acetonitrile/water (1:1).

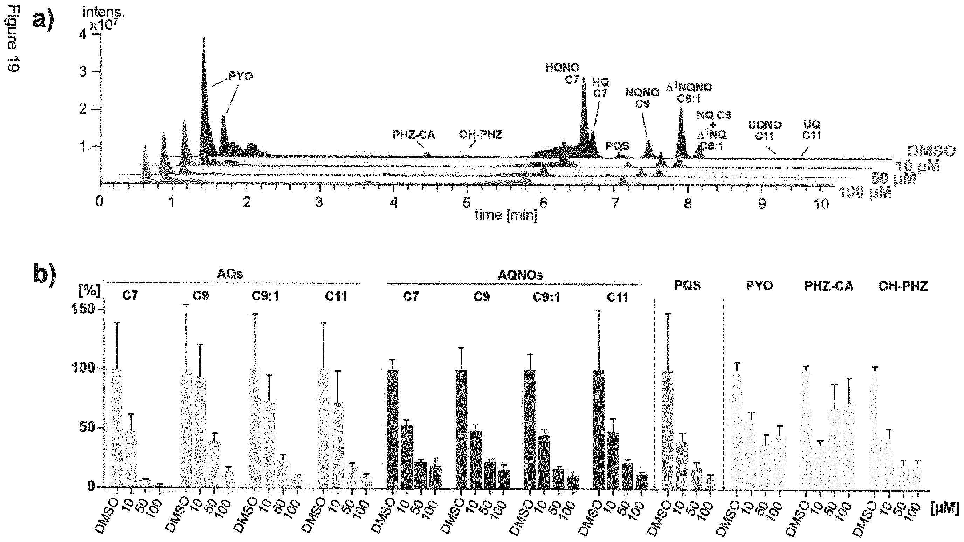

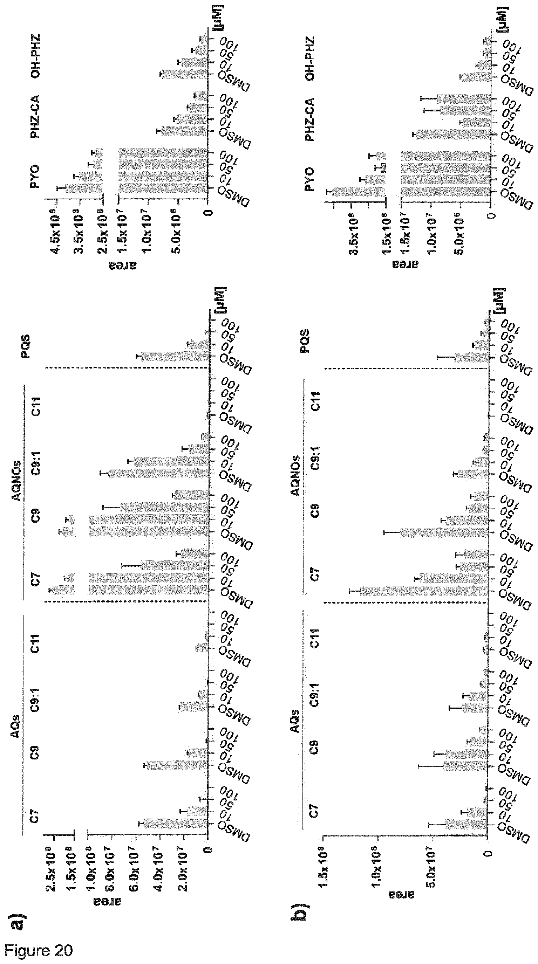

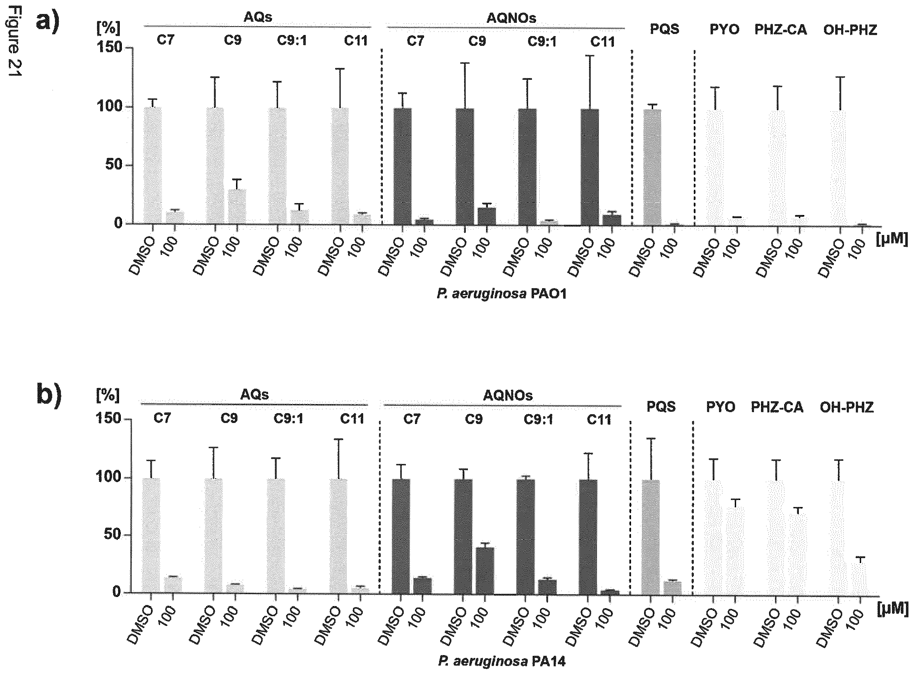

Increasing concentrations of the PqsD inhibitor considerably decreased the intensity of quinolones and phenazines in both strains (Figure 17b, Figure 19a). Quantification of individual quinolones revealed a global down-regulation of AQs and AQNOs. The production of all major quorum sensing signals, PQS and the chain length congeners of HHQ was completely inhibited in *P*. *aeruginosa* PAO1 between 50 µM and 100 µM with an average IC₅₀ < 10 µM and to a slightly lesser degree in *P. aeruginosa* PA14 (Figures 17c, 19b and 20). Inhibition of quinolone signal production consequently caused the down-regulation of phenazines, which was strongest for 1-OH-PHZ and weakest for pyocyanin. Finally, also production of the different quinolone N-oxide congeners was inhibited by the compound represented by Formula (2) in dose response manner including the three major *N*-oxides HQNO, NQNO and *trans*-Δ¹-NQNO. Inhibition of quinolone production by the compound represented by Formula (5) has also been observed (Figure 21).

### Example 6: 2-ABA feeding of P. aeruginosa PAO1

10 µL of a 5 mM stock of the compound represented by Formula (2) in DMSO or 10 µL of DMSO as vehicle control (1 % final DMSO concentration) was added in 1 mL LB medium in 15 mL polypropylene centrifugal tubes. Then, 5 µL *P*. *aeruginosa* PAO1 overnight culture was added and grown at 37 °C (190 rpm). Due to the instability of 2-ABA, it was added in six portions. After several time points (6h, 6.5h, 7h, 7.5h, 8h and 10h) the cultures treated with the compound represented by Formula (2) were supplemented with 20 µL 100 mM 2-ABA stock in basic solution (1% NH₄OH) or 20 µL basic solution (1% NH₄OH) as vehicle control. To investigate the effect of basic solution, the DMSO containing control samples were supplemented with same amounts of basic solution compared to treated samples. The total amount of 2-ABA added to each sample was 12 mM and the pH of the cultures after addition of the total volume of basic solution was under pH 8. After 11 h cultures were centrifuged, supernatants sterile filtered and HHQ extracted and quantified by LC-MS/MS according to the previously described protocol.

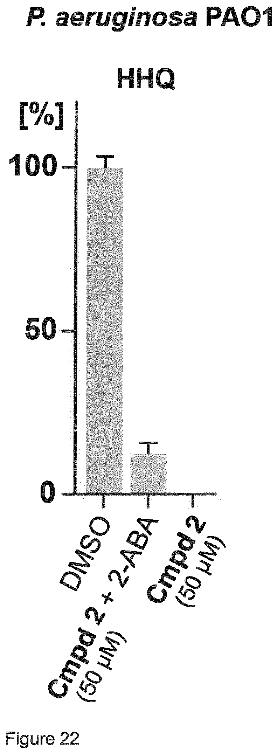

By the external addition of synthetic 2-ABA (Drees, S. L.; Li, C.; Prasetya, F.; Saleem, M.; Dreveny, I.; Williams, P.; Hennecke, U.; Emsley, J.; Fetzner, S., J Biol Chem 2016, 291 (13), 6610-24) to a culture of *P*. *aeruginosa* PAO1 grown with 50 µM of compound of Formula (2) it was shown that HHQ production can be partially restored (Figure 22).

The results with *P*. *aeruginosa* confirm that compound for use according to the present invention as highly active inhibitor with unprecedented efficacy in the global inhibition of quinolone biosynthesis and down-regulation of phenazine production. The compound for use according to the present invention is the most potent *in situ* PqsD inhibitor reported so far that causes global inhibition of quinolone biosynthesis. Thus, the compound for use according to the present invention can be used as an inhibitor of virulence (patho-blocker) for example for treating chronic infections or can be used in combination with other anti-bacterial agents, such as antibiotics, to increase sensitivity of the bacteria, such as bacteria mediating pathogenicity by quinolone dependent production of virulence factors, as e.g. *Pseudomonas aeruginosa* and species of the genus *Burkholderia*, for treatment of multiresistant strains in mammals.

## Claims

1. A compound for use in the treatment of infections with pathogenic bacteria in vertebrates, wherein the compound is represented by the general Formula (1) or a pharmaceutically acceptable salt thereof 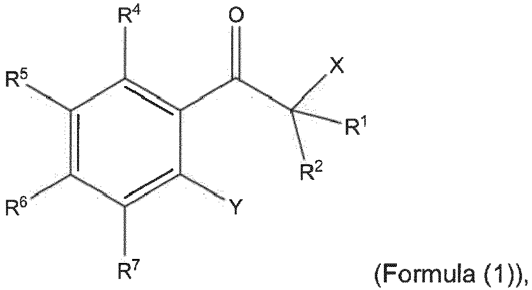 wherein
X is a halogen atom;
Y is NHR³, a hydrogen atom, or a halogen atom;
R¹ and R² are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group;
R³, when present, is selected from the group consisting of a hydrogen atom, a substituted or unsubstituted alkyl group having from 1 to 11 carbon atoms, a substituted or unsubstituted cycloalkyl group having from 4 to 12 carbon atoms, a substituted or unsubstituted alkenyl group having from 2 to 11 carbon atoms, a substituted or unsubstituted cycloalkenyl group having from 4 to 12 carbon atoms, a substituted or unsubstituted alkynyl group having from 2 to 11 carbon atoms, a substituted or unsubstituted aryl group, and a substituted or unsubstituted heteroaryl group, wherein R³ may bind to R⁷ to form a ring; and
R⁴ to R⁷ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, a substituted or unsubstituted heteroaryl group, a halogen atom, -NE¹E², -NO₂, -CN, -OE³, -SE⁴, -C(O)E⁵, -C(O)NE⁶E⁷, - COOE⁸, and -SO₃E⁹, wherein E¹ to E⁹ are each independently selected from the group consisting of a hydrogen atom, a substituted or unsubstituted alkyl group, a substituted or unsubstituted cycloalkyl group, a substituted or unsubstituted alkenyl group, a substituted or unsubstituted cycloalkenyl group, a substituted or unsubstituted alkynyl group, a substituted or unsubstituted aryl group, and a substituted or unsubstituted heteroaryl group, and wherein two or more of R⁴ to R⁷ may bind to each other to form one or more rings.

2. The compound for use according to claim 1, wherein X is a bromine atom or a chlorine atom.

3. The compound for use according to claim 1 or 2, wherein R¹ and R² are each independently selected from the group consisting of a hydrogen atom, a substituted or unsubstituted alkyl group, and a substituted or unsubstituted alkenyl group.

4. The compound for use according to claim 3, wherein. R¹ and R² are each a hydrogen atom

5. The compound for use according to any one of claims 1 to 4, wherein R⁴ to R⁷ are each independently selected from the group consisting of a hydrogen atom, a substituted or unsubstituted alkyl group, a substituted or unsubstituted alkenyl group, -OE³, and a halogen atom; and two or more of R⁴ to R⁷ may bind to each other to form one or more saturated or unsaturated, 5- or 6-membered rings. Preferably, the rings are

6. The compound for use according to claim 5, wherein R⁴ to R⁷ are each a hydrogen atom.

7. The compound for use according to any one of claims 1 to 6, wherein Y is NHR³.

8. The compound for use according to any one of claims 1 to 7, wherein R³, when present, is selected from the group consisting of a hydrogen atom, a substituted or unsubstituted alkyl group having from 1 to 11 carbon atoms, and a substituted or unsubstituted alkenyl group having from 2 to 11 carbon atoms; and R³ may bind to R⁷ to form a saturated or unsaturated 5- or 6-membered ring.

9. The compound for use according to any one of claims 1 to 5, wherein the compound is selected from the group consisting of the compounds represented by the following Formulas (2) to (7), or a pharmaceutically acceptable salt thereof.

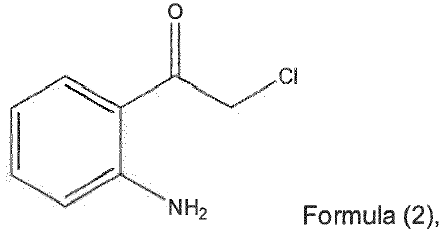

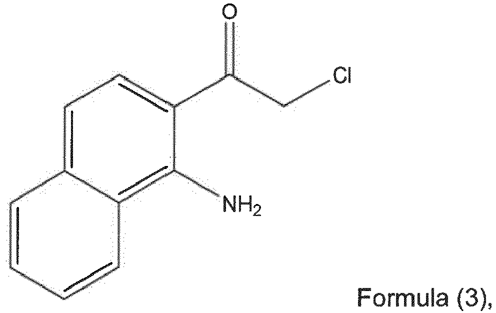

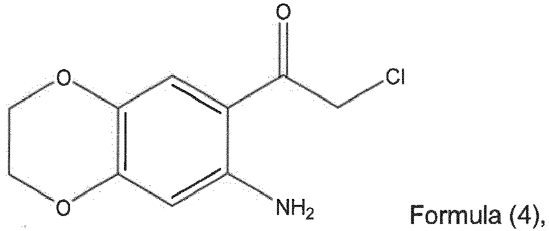

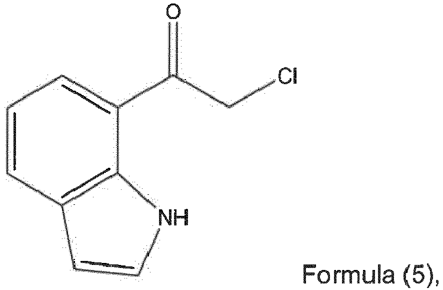

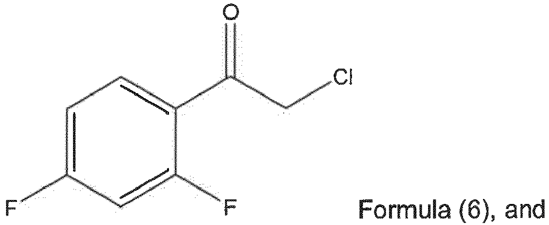

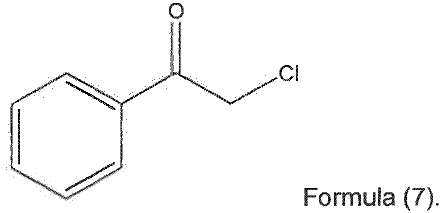

10. The compound for use according to any one of claims 1 to 9, wherein the pathogenic bacteria are *Pseudomonas aeruginosa* and species of the genus *Burkholderia.*

11. The compound for use according to any one of claims 1 to 10, wherein the compound is used in the treatment of an infection in a mammal, wherein the infection is selected from the group consisting of sepsis, wound infections, endocarditis, meningitis, and chronic respiratory infections in cystic fibrosis patients.

12. The compound for use according to claim 11, wherein the mammal is a human.

13. A pharmaceutical composition comprising the compound as defined in any one of claims 1 to 9 in a pharmaceutically active amount, and optionally a pharmaceutically acceptable carrier, excipient or diluent.

14. The pharmaceutical composition according to claim 13 for use in the treatment of infections with pathogenic bacteria, preferably selected from *Pseudomonas aeruginosa* and species of the genus *Burkholderia,* in a vertebrate, preferably a human.

15. The pharmaceutical composition according to claim 13 or 14, wherein the infection is selected from the group consisting of sepsis, wound infections, endocarditis, meningitis, and chronic respiratory infections in cystic fibrosis patients.
